(19) Europäisches Patentamt
European Patent Office
Office européen des brevets

(11) Numéro de publication: **0 158 545**
**B1**

(12) # FASCICULE DE BREVET EUROPEEN

(45) Date de publication du fascicule du brevet: **22.04.87**

(51) Int. Cl.[4]: **C 07 C 149/00**, C 07 C 147/00, A 61 K 31/00

(21) Numéro de dépôt: **85400431.4**

(22) Date de dépôt: **06.03.85**

(54) **Dérivés d'acide [alpha-(alkylaminométhyl)-benzylthio]-acétique, procédé de préparation et utilisation en thérapeutique.**

(30) Priorité: **23.03.84 FR 8404543**

(43) Date de publication de la demande:
**16.10.85 Bulletin 85/42**

(45) Mention de la délivrance du brevet:
**22.04.87 Bulletin 87/17**

(84) Etats contractants désignés:
**AT BE CH DE FR GB IT LI LU NL SE**

(56) *Documents cités:*
**FR - A - 2 326 181**
**FR - A - 2 385 693**

**CHEMICAL ABSTRACTS, volume 88, no. 17, 24 avril 1978, page 507, abrégé 120743h, Columbus, Ohio, US; D. ALEKSIEV: "Nucleophilic addition of thioglycolic acid to conjugated nitroalkenes"**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Clisci, Serge et al, S.A. FEDIT-LORIOT CONSEILS EN PROPRIETE INDUSTRIELLE 38, avenue Hoche, F-75008 Paris (FR)**

**EP 0 158 545 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne, en tant que produits industriels, de nouveaux dérivés d'acide [α-(alkylaminométhyl)-benzylthio]-acétique. Elle concerne également le procédé de préparation et l'utilisation en thérapeutique de ces nouveaux produits.

Les dérivés d'acide [α-(alkylaminométhyl)-benzylthio]-acétique selon l'invention, à savoir les amides, les esters et les acides hydroxamiques de formule I ci-après, sont utiles en thérapeutique notamment en raison de leurs propriétés sur le système nerveux central (SNC) et plus particulièrement en tant qu'agents antidépresseurs.

Les nouveaux produits selon l'invention qui sont des dérivés aminés de l'acide (benzylthio) acétique sont caractérisés en ce qu'ils sont choisis parmi l'ensemble constitué par:

a) les dérivés d'acide [α-(alkylaminométhyl)-benzylthio]-acétique de formule générale:

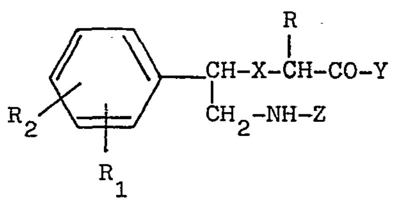

(I)

dans laquelle,

X est S ou SO,

Y est un groupe alkoxy en $C_1$-$C_4$, un groupe $NH_2$ ou un groupe NHOH,

Z est un groupe alkyle en $C_1$-$C_4$,

R est H ou $CH_3$,

$R_1$ et $R_2$, identiques ou différents, représentent chacun H, F, Cl ou Br;

b) leurs isomères optiques, et

c) leurs sels d'addition.

Par sels d'addition, on entend ici les sels d'addition d'acides obtenus par réaction d'une base libre de formule I avec un acide minéral ou organique, et les sels d'ammonium. Parmi les acides utilisables pour salifier les bases de formule I, on peut notamment citer les acides chlorhydrique, bromhydrique, nitrique, sulfurique, acétique, propionique, oxalique, fumarique, maléique, succinique, benzoïque, cinnamique, mandélique, citrique, malique, tartrique, p-toluènesulfonique et méthanesulfonique. Parmi les composés permettant d'obtenir des sels d'ammonium, on peut citer notamment $ICH_3$ et $ClCH_3$. Les sels d'addition d'acides sont les sels préférés, et, parmi ces derniers, les plus intéressants sont les chlorhydrates.

Parmi les groupes alkoxy en $C_1$-$C_4$ intervenant dans la définition du radical Y, qui conviennent selon l'invention, on peut notamment mentionner les groupes méthyloxy, éthyloxy, n-propyloxy, i-propyloxy, n-butyloxy, i-butyloxy et t-butyloxy.

Parmi les groupes alkyle en $C_1$-$C_4$, qui interviennent dans la définition du radical Z et qui conviennent selon l'invention, on peut notamment citer les groupes $CH_3$, $CH_2CH_3$, $CH_2CH_2CH_3$, $CH(CH_3)_2$, $CH_2CH_2CH_2CH_3$, $CH_2CH(CH_3)_2$, $CH(CH_3)CH_2CH_3$ et $C(CH_3)_3$, les groupes alkyle préférés du point de vue thérapeutique étant les groupes isopropyle et tertiobutyle.

L'invention concerne donc (i) les esters (Y = alkoxy en $C_1$-$C_4$), les amides (Y = $NH_2$) et les acides hydroxamiques (Y = NHOH), (ii) les isomères optiques résultant de la sépartion des racémiques précédents, et (iii) les sels d'addition. Ces produits sont utiles en thérapeutique comme indiqué ci-dessus, les esters sont également utiles en tant que composés intermédiaires de synthèse intervenant dans la préparation des amides et des acides hydroxamiques. D'une manière générale tous les composés selon l'invention présentent la propriété commune d'agir sur le SNC en tant qu'agents antidépresseurs. Certains composés, notamment ceux des exemples 9 (CRL 41 250) et 10 (CRL 41 252), possèdent en outre des propriétés cardiovasculaires intéressantes, en particulier des effets vasodilatateurs bénéfiques.

Un certain nombre de composés selon l'invention a été consigné de façon nullement limitative dans le tableau I ci-après. Les composés les plus intéressants sur le plan thérapeutique sont les amides (Y = $NH_2$) et les acides hydroxamiques (Y = NHOH). Parmi ceux-ci on préfère, en raison de leurs effets antidépresseurs, (i) les racémiques et isomères énantiomères de [α-(tertiobutylaminométhyl)-benzylthio]-acétamide, et de l'acide [α-(tertiobutylaminométhyl)-benzylthio]-acétohydroxamique et (ii) leurs sels d'addition.

Les composés de formule I selon l'invention peuvent être préparés selon une méthode connue en soi par application de mécanismes réactionnels classiques, notamment selon le diagramme A ci-après [où Ar représente un groupe $(R_1R_2)C_6H_3$ dans lequel $R_1$ et $R_2$ sont définis comme indiqué ci-dessus, Hal est un atome d'halogène (notamment F, Cl ou Br), A est un groupe alkoxy en $C_1$-$C_4$, et Z est défini comme indiqué ci-dessus].

Le procédé de préparation d'un composé de formule I que l'on préconise selon l'invention est caractérisé en ce que:

a) l'on fait réagir une N-alkyl-N-(2-halogéno-2-phényléthyl)-amine de formule:

$$Ar-\underset{\displaystyle Hal}{\underset{|}{CH}}-CH_2-NH-Z \qquad (II)$$

où Hal est un atome d'halogène (notamment F, Cl ou Br, et de préférence Cl), et Z et Ar sont définis comme indiqué ci-dessus, avec un thioglycolate d'alkyle de formule:

$$HS-CHR-CO-A \qquad (III)$$

où A est un groupe alkoxy en $C_1$-$C_4$ et R est défini comme indiqué ci-dessus, dans un alcool de formule:

$$H-A \qquad (IV)$$

où A est défini comme ci-dessus, en présence d'un métal alcalin (notamment Na ou K), à une température comprise entre la température ambiante (15-20°C) et la température de reflux du milieu

réactionnel, pour obtenir un [α-(alkylaminométhyl)-benzylthio]-acétate d'alkyle de formule:

Ar–CH–S–CHR–CO–A (V)
    |
    $CH_2$–NH–Z

b) si nécessaire, on fait réagir, pendant au moins 5 h à une température comprise entre 15 et 25° C, le composé [α-(alkylaminométhyl)-benzylthio]-acétate d'alkyle ainsi obtenu avec un composé choisi parmi l'ensemble constitué par $NH_3$ et $NH_2OH$ pour obtenir l'amide, de formule:

Ar–CH–S–CHR–CO–$NH_2$ (VI)
    |
    $CH_2$–NH–Z

et respectivement l'acide hydroxamique de formule:

Ar–CH–S–CHR–CO–NHOH (VII)
    |
    $CH_2$–NH–Z

correspondants, et

c) si nécessaire, on soumet le dérivé soufré choisi parmi l'ensemble constitué par les composés de formule V, VI et VII à une réaction d'oxydation au moyen de $H_2O_2$, pour obtenir le dérivé sulfinyle (X = SO) correspondant.

De façon avantageuse, la réaction du stade a) est mise en œuvre en faisant réagir 1 mole de II avec 1 mole de III en présence de 2 atomes-grammes de métal alcalin (sous forme d'alcoolate Na-A ou K-A) pendant au moins 1 h à la température de reflux du mélange réactionnel, ou pendant 6-12 h à 15-20° C.

La réaction d'amidification du stade b) est mise en œuvre en faisant réagir 1 mole d'ester V avec 2 à 10 moles de $NH_3$ dissous dans un alcool H-A ou de l'eau, pendant 5 à 50 h, à une température comprise entre 15 et 25° C.

La réaction selon le stade b) de l'ester V avec l'hydroxylamine pour obtenir l'acide hydroxamique VII est avantageusement réalisée en faisant réagir, à une température comprise entre 15 et 25° C, pendant 5 à 20 h, 1 mole d'ester V avec 1 à 1,2 mole de $NH_2OH$.

La réaction d'oxydation du stade c) est réalisée par mise en contact d'un composé de formule V, VI ou VII avec $H_2O_2$ en présence d'acide acétique. De façon avantageuse on fait réagir 1 ml d'eau oxygénée à 110-130 volumes avec 0,01 mole de V, VI ou VII à une température inférieure ou égale à 50° C; de préférence on opérera à 40-45° C pendant 1 h puis à la température ambiante (15-20° C) pendant 5 à 15 h.

Les isomères optiques lévogyre et dextrogyre sont séparés du racémique correspondant selon une méthode connue en soi, notamment par cristallisation fractionnée.

Selon l'invention on préconise une composition thérapeutique qui est caractérisée en ce qu'elle renferme, en association avec un excipient physiologique acceptable, au moins un composé choisi parmi l'ensemble constitué par les racémiques, lévogyres et dextroyres de formule I ci-dessus, d'une part, et leurs sels d'addition, d'autre part.

Bien entendu, dans une telle composition le principe actif intervient en quantité pharmaceutiquement efficace.

D'autres avantages et caractéristiques de l'invention seront mieux compris à la lecture qui va suivre d'exemples de préparation et de résultats d'essais neuropsychopharmacologiques. L'ensemble de ces éléments n'est pas limitatif mais donné à titre d'illustration.

*Préparation I:*

*Obtention du chlorhydrate de [α-(tertiobutylaminométhyl)-benzylthio]-acétamide*
(Exemple 1; N° de Code: CRL 41 190)

On ajoute à froid 0,08 mole (8 ml) de thioglycolate de méthyle dans une solution de méthylate de sodium obtenue à partir de Na (3,7 g; 0,16 Atg) et de $CH_3OH$ (100 ml). On coule ensuite à 20° C dans le mélange résultant 20 g (0,08 mole) de 2-tertiobutylamino-1-phényl-1-chloroéthane ($F_{inst}$ = 204-206° C) en solution dans 50 ml de méthanol, puis chauffe 1 h à reflux. On écarte par filtration le NaCl formé et évapore le filtrat sous vide. On reprend le résidu d'évaporation avec 100 ml d'éther diéthylique, lave à l'eau, extrait avec HCl 3N, précipite à froid avec NaOH concentrée, extrait à l'éther, sèche puis évapore l'éther. Le résidu d'évaporation (qui se présente sous la forme d'une huile et est essentiellement constitué par l'[α-(tertiobuylaminométhyl)-benzylthio]-acétate d'éthyle) est repris avec 50 ml de $CH_3OH$ et versé dans 100 ml de méthanol ammoniacal (mélange renfermant 100 g/l de $NH_3$); après 48 h de contact à 15-20° C on évapore $CH_3OH$ sous vide, reprend à l'éther, filtre sur charbon puis précipite le chlorhydrate attendu au moyen d'éthanol chlorhydrique. Après essorage et recristallisation dans l'éthanol on obtient, avec un rendement de 75%, le chlorhydrate de [α-(tertiobutylaminométhyl)-benzylthio]-acétamide. $F_{inst}$ = 154-155° C (avec décomposition).

*Préparation II:*

*Obtention de [α-(tertiobutylaminométhyl)-benzylsulfinyl]-acétamide*
(Exemple 2; N° de Code: CRL 41 191)

6,05 g (0,02 mole) de chlorhydrate de [α-(tertiobutylamino-méthyl)- (CRL 41 190) dans 20 ml d'acide acétique sont oxydés avec 2 ml d'eau oxygénée à 110 volumes. Après 1 h à 40° C et une nuit à 20° C on évapore l'acide acétique sous vide, reprend avec 30 ml d'eau, précipite la base à froid avec NaOH concentrée, essore, lave avec 5 ml d'eau froide puis 10 ml d'éther, et sèche. Par recristallisation dans l'acétate d'éthyle, on obtient, avec un rendement de 64%, le CRL 41 191. $F_{inst}$ = 147-148° C.

*Préparation III:*

*Obtention du chlorhydrate de l'acide [α-(tertiobutylaminométhyl)-benzylthio]-acétohydroxamique*
(Exemple 3; N° de Code: CRL 41 214)

4,35 g (0,062 mole) de chlorhydrate d'hydroxylamine en solution dans 50 ml de méthanol anhydre sont ajoutés à une soluiton de 2,8 g (0,12 Atg) de sodium dans 100 ml de méthanol anhydre. On filtre le chlorure de sodium formé et ajoute au filtrat 16 g (0,057 mole) de [α-(tertiobutylaminométhyl)-benzylthio]-acétate de méthyle, après une nuit en contact à 20° C on évapore à sec sous vide. On reprend le résidu d'évaporation avec 30 ml d'eau, additionne goutte à goutte HCl 3N jusqu'à un pH compris entre 8 et 9, extrait avec $CH_2Cl_2$, lave avec 2 × 10 ml d'eau, sèche et évapore sous vide. Le résidu d'évaporation est repris avec 100 ml d'acétate d'éthyle et acidifié avec de l'éthanol chlorhydrique pour précipiter le chlorhydrate attendu. On essore, lave à l'acétone et par recristallisation dans l'éthanol, on obtient, avec un rendement de 72%, le CRL 41 214. $F_{inst}$ = 198-200° C (avec décomposition).

*Préparation IV:*

*Obtention du fumarate de [α-(isopropylaminométhyl)-benzylthio]-acétate de méthyle*

$$C_6H_5-CH-S-CH_2-CO_2-CH_3 \quad HOOC-CH$$
$$| \quad , \quad ||$$
$$CH_2-NH-CH(CH_3)_2 \quad HOOC-CH$$

(Exemple 4; N° de Code: CRL 41 215)

On prépare une solution de méthylate de sodium avec 4,6 g (0,2 Atg) de sodium et 150 ml de méthanol, on ajoute 10 ml (0,1 mole) de thioglycolate de méthyle puis goutte à goutte, à reflux en agitant, une solution de 23,4 g (0,1 mole) de chlorhydrate de 2-isopropylamino-1-phényl-1-chloroéthane ($F_{inst}$ = 183-184° C) dans 70 ml de méthanol. On maintient encore 2 h à reflux, filtre le précipité, évapore le filtrat à sec sous vide. Le résidu d'évaporation est repris avec 200 ml d'éther, lavé avec 2 × 50 ml d'eau et extrait avec 50 ml d'HCl 3N. La base est précipitée à froid avec NaOH concentrée. On extrait à l'éther, sèche et évapore. L'huile ainsi obtenue est mise en solution dans 100 ml d'éthanol et acidifiée avec 9,3 g (0,08 mole) d'acide fumarique. On filtre le mélange résultant à chaud, refroidit et essore. Par recristallisation dans le méthanol, on obtient, avec un rendement de 62%, le CRL 41 215. $F_{inst}$ = 138-139° C.

*Préparation V:*

*Obtention du chlorhydrate de [α-(isopropylaminométhyl)-benzylthio]-acétamide*
(Exemple 5; N° de Code: CRL 41 216)

12,1 g (0,045 mole) d'[α-isopropylaminométhyl)-benzylthio]-acétate de méthyle sont agités à 20° C pendant 16 h avec 100 ml d'ammoniaque (renfermant 200 g/l de $NH_3$). On extrait à l'éther, lave à l'eau (2 × 20 ml), sèche et évapore à sec sous vide. On reprend le résidu huileux d'évaporation avec 50 ml d'acétate d'éthyle, acidifie avec de l'éthanol chlorhydrique et essore. Par recristallisation dans l'éthanol, on obtient, avec un rendement de 58%, le CRL 41 216. $F_{inst}$ = 118-120° C.

*Préparation VI:*

*Obtention du fumarate de 2-{[α-(tertiobutylaminométhyl)-benzyl]-thio}-propionamide*

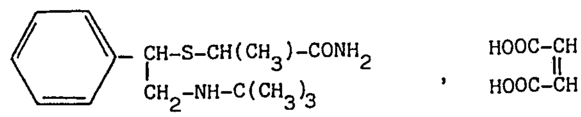

(Exemple 9: N° de Code: CRL 41 250)

On prépare une solution de méthylate de sodium avec 3,22 g (0,14 Atg) de sodium et 100 ml de méthanol, on ajoute 8,4 g (0,07 mole) de thiolactate de méthyle puis 17,4 g (0,07 mole) de chlorhydrate de 2-tertiobutylamino-1-phényl-1-chloroéthane. On agite le milieu réactionnel résultant pendant 5 h à une température comprise entre 0 et 5° C, puis pendant 12 h à 20° C; on écarte par filtration le précipité formé, évapore l'alcool sous vide, reprend avec 100 ml d'éther, lave la solution éthérée avec 3 fois 50 ml d'eau, sèche sur $MgSO_4$, évapore l'éther sous vide, verse le résidu d'évaporation dans 100 ml d'ammoniaque à 20% et agite le mélange résultant pendant 12 h. On extrait à l'éther, lave à l'eau, extrait avec 100 ml d'HCl 2N, reprécipite la base libre au moyen de $NaHCO_3$, extrait à l'éther, sèche et évapore sous vide. Le résidu d'évaporation (13 g), qui est constitué par le 2-{[α-(tertiobutylaminométhyl)-benzyl]-thio}-propionamide (produit de l'exemple 12), mis en suspension dans 40 ml d'eau, est acidifié avec 5,8 g (0,05 mole) d'acide fumarique. On filtre à chaud, refroidit, essore. Par recristallisation dans l'eau, on obtient, avec un rendement de 38%, le CRL 41 250. $F_{inst}$ = 156-157° C.

*Préparation VII:*

*Obtention du fumarate de [α-(tertiobutylaminométhyl)-2-fluorobenzylthio]-acétamide*
(Exemple 10; N° de Code: 41 252)

Dans une solution froide de 4,6 g de méthylate de sodium (obtenue à partir de 0,2 Atg de Na et de 150 ml de $CH_3OH$), on ajoute 10 ml (0,1 mole) de thioglycolate de méthyle et 26,6 g (0,1 mole) de chlorhydrate de 1-(2-fluorophényl)-2-tertiobutylamino-1-chloroéthane ($F_{inst}$ = 202-203° C avec décomposition). On agite 0,5 h à 20° C et 3 h à reflux. On refroidit, écarte par filtration le NaCl formé, évapore le filtrat sous vide (pour distiller l'alcool), reprend à l'éther, lave trois fois à l'eau, sèche, évapore puis traite le résidu d'évaporation huileux avec 300 ml de $NH_4OH$ à 28% en agitant pendant 16 h. On extrait à l'éther, lave à l'eau, extrait avec 100 ml d'HCl 2N, précipite avec NaOH concentrée, extrait à l'éther, lave à l'eau, sèche et évapore. Le résidu d'évaporation huileux (23,5 g) qui est constitué par l'[α-(tertiobutylaminométhyl)-2-fluorobenzylthio]-acétamide (produit de l'exemple 13), mis en suspension dans 40 ml d'eau, est acidifié avec 9,8 g d'acide fumarique. On filtre à chaud, refroidit, essore et sèche. Par recristallisation dans le mélange éthanol-acétate d'éthyle (4:1) v/v, on obtient, avec un rendement de 58%, le CRL 41 252. $F_{inst}$ = 118° C.

En procédant comme indiqué dans la préparation VII ci-dessus, mais en remplaçant le 1-(2-fluorophényl)-2-tertiobutylamino-1-chloroéthane par le 1-(3,4-dichlorophényl)-2-isopropylamino-1-chloroéthane ($F_{inst}$ = 194-195°C), on obtient l'[α-(isopropylaminométhyl)-3,4-dichlorobenzylthio]-acétamide (produit de l'exemple 14), puis le fumarate de [α-(isopropylaminométhyl)-3,4-dichlorobenzylthio]-acétamide (produit de l'exemple 11; N° de code: CRL 41 253) après recristallisation finale dans le méthanol. $F_{inst}$ = 158°C.

On a résumé ci-après les résultats des essais neuropsychopharmacologiques qui ont été entrepris avec les produits préférés selon l'invention, le CRL 41 190 (Ex. 1) et le CRL 41 214 (Ex. 3). Dans ce résumé, les deux produits à tester mis en solution dans de l'eau distillée ont été administrés, sauf indication contraire, par voie intrapéritonéale sous un volume de 20 ml/kg chez la souris mâle et sous un volume de 5 ml/kg chez le rat mâle.

*Essais relatifs au CRL 41 190*

A. *Toxicité*

La DL-0 (dose maximale non mortelle) du CRL 41 190 est supérieure à 128 mg/kg par voie I.P. chez la souris mâle, et la DL-30 est de l'ordre de 256 mg/kg.

B. *Comportement global et réactivités*

Des lots de trois animaux sont observés avant puis 0,25 h, 0,5 h, 1 h, 2 h, 3 h et 24 h après administration de CRL 41 190. On observe ce qui suit:

1. *Chez la souris:*
   – *à la dose de 1 mg/kg:* aucune modification nette du comportement et des réactivités;
   – *aux doses de 4 et 16 mg/kg:* une augmentation de la réaction de peur, d'une part, et une augmentation de la réactivité au toucher pendant 0,5 h, d'autre part;
   – *à la dose de 64 mg/kg:* une augmentation de la réaction de peur pendant 1 h et de la réactivité au toucher de 0,5 h à 1 h, une sédation, et une mydriase faible pendant 1 h.
2. *Chez le rat:*
   – *aux doses de 0,5 mg/kg à 8 mg/kg:* un comportement, des réactivités, une variation de la température rectale, et une variation du diamètre pupillaire sensiblement comparables à ceux du lot témoin;
   – *à la dose de 32 mg/kg:* une mydriase pendant 1 h.

C. *Interaction avec l'apomorphine*

1. *Chez la souris:*
   Des lots de 6 souris reçoivent le produit à étudier 0,50 h avant une injection sous-cutanée de 1 mg/kg ou 16 mg/kg d'apomorphine. On observe que, à la plus forte dose utilisée (64 mg/kg), le CRL 41 190 s'oppose à l'hypothermie induite par 1 mg/kg d'apomorphine. Par contre, le CRL 41 190 antagonise dès la dose de 1 mg/kg l'hypothermie induite par 16 mg/kg d'apomorphine. Par ailleurs, on constate que les comportements de verticalisation et les stéréotypies ne sont pratiquement pas modifiés.
2. *Chez le rat:*
   Le CRL 41 190 est administré à des lots de 6 rats, 0,50 h avant l'injection sous-cutanée de 0,5 mg/kg d'apomorphine. On observe que le CRL 41 190 laisse inchangées les stéréotypies induites par l'apomorphine.

D. *Interaction avec l'amphétamine*

L'amphétamine (2 mg/kg) est injectée par voie intrapéritonéale à des lots de 6 rats, une demi-heure après l'administration de CRL 41 190.

On constate que, aux doses de 8 et 32 mg/kg, le CRL 41 190 augmente nettement la durée des stéréotypies amphétaminiques.

E. *Interaction avec la réserpine*

Quatre heures après l'injection intrapéritonéale de 2,5 mg/kg de réserpine, des lots de 12 souris reçoivent le CRL 41 190. On étudie l'influence du produit à tester sur l'hypothermie et le ptôsis réserpiniques.

On observe que, dès la dose de 4 mg/kg, le CRL 41 190 antagonise l'hypothermie réserpinique. Cet antagonisme est très net aux deux plus fortes doses utilisées (16 mg/kg et 64 mg/kg).

On observe également que, aux deux plus fortes doses utilisées (16 mg/kg et 64 mg/kg), le CRL 41 190 antagonise faiblement le ptôsis réserpinique à partir de la demi-heure qui suit son administration.

F. *Interaction avec l'oxotrémorine*

Le CRL 41 190 est administré à des lots de 6 souris une demi-heure avant l'injection intrapéritonéale de 0,5 mg/kg d'oxotrémorine.

1. *Action sur la température:*
   On constate que, dès la dose de 1 mg/kg, le CRL 41 190 s'oppose à l'hypothermie induite par l'oxotrémorine. Cet antagonisme est net aux doses de 4 mg/kg, 16 mg/kg et surtout 64 mg/kg.
2. *Action sur les tremblements:*
   On constate que, à la dose de 64 mg/kg, le CRL 41 190 diminue faiblement l'intensité des tremblements dus à l'oxotrémorine.
3. *Action sur les symptômes cholinergiques périphériques*
   On note que, à la dose de 64 mg/kg, le CRL 41 190 diminue faiblement la salivation entre les 1,5 h et 2 h qui suivent son administration. On note également que, aux autres doses, le CRL 41 190 ne modifie pratiquement pas les signes de stimulation cholinergique périphérique dus à l'oxotrémorine.

G. *Action sur le test des quatre plaques, la traction et l'électrochoc*

Le test est pratiqué sur des lots de 10 souris 0,50 h après l'administration du produit à étudier.

On observe que le CRL 41 190 n'entraîne pas d'augmentation du nombre de passages punis, qu'il ne provoque pas d'incapacité motrice majeure et ne modifie pas les effets convulsivants, mais qu'il aggrave, à la dose de 64 mg/kg, les effets létaux de l'électrochoc.

H. *Action sur la motilité spontanée*

0,5 h après avoir reçu le CRL 41 190, les souris (12 animaux par dose, 24 animaux témoins) sont placés en actimètre où leur motilité est enregistrée pendant 30 minutes.

On observe que, à la plus forte dose utilisée (64 mg/kg), le CRL 41 190 ne modifie pratiquement pas l'activité motrice spontanée de la souris.

I. *Action sur l'agressivité intergroupes*

Après avoir séjourné pendant 3 semaines dans chacune des moitiés d'une cage séparées par une cloison opaque, des groupes de 3 souris reçoivent le CRL 41 190. Une demi-heure plus tard, les deux groupes d'une même cage sont réunis par retrait de la cloison et on note le nombre de combats qui surviennent en 10 minutes.

On observe que, à la plus forte dose utilisée (64 mg/kg), le CRL 41 190 augmente modérément le nombre de combats.

J. *Action vis-à-vis de quelques comportements perturbés par divers agents*

1. *Motilité réduite par habituation à l'enceinte:*
   Après 18 heures de séjour dans les actimètres, les souris (6 par dose, 12 témoins) reçoivent le CRL 41 190. Elles sont aussitôt replacées dans leurs enceintes respectives et, une demi-heure plus tard, on enregistre leur motilité pendant 30 minutes.
   On constate que le CRL 41 190 n'entraîne pas de reprise d'activité motrice chez la souris habituée à son enceinte.
2. *Motilité réduite par agression hypoxique:*
   Une demi-heure après avoir reçu le CRL 41 190, les souris (10 par dose, 20 témoins) sont soumises à une anoxie hypobare aiguë [dépression de 600 mmHg (i.e. environ 8 × $10^4$ pascals) en 90 secondes, puis détente de 45 secondes], puis elles sont placées en actimètres où leur motilité est enregistrée pendant 10 minutes.
   On observe que le CRL 41 190 n'entraîne pas d'amélioration de la récupération motrice chez la souris dont la motilité a été déprimée à la suite d'un séjour bref dans une enceinte à pression réduite. A la plus forte dose utilisée (64 mg/kg) une mortalité (3/10) est observée immédiatement après l'hypoxie aiguë.
3. *Anoxie asphyxique:*
   Des lots de 10 souris reçoivent le CRL 41 190 une demi-heure avant l'administration intrapéritonéale de 32 mg/kg d'un agent curarisant de référence, le triiodoéthylate de gallamine.
   On observe que le CRL 41 190 ne modifie pas le délai d'apparition des convulsions et de la mort consécutives à une anoxie asphyxique provoquée par un curarisant.

K. *Interaction avec le barbital*

Une demi-heure après l'administration de CRL 41 190, des lots de 10 souris reçoivent une injection intrapéritonéale de barbital (220 mg/kg).

On observe que, à la plus forte dose utilisée (64 mg/kg), le CRL 41 190 diminue la durée du sommeil barbiturique.

L. *Action sur le «désespoir comportemental»*

Une demi-heure après avoir reçu le CRL 41 190, des lots de 6 souris sont placés dans un becher rempli d'eau sur une hauteur de 6 cm. On note la durée totale d'immobilité entre la 2[e] et la 6[e] minute suivant l'immersion.

On observe que, à la plus forte dose utilisée (64 mg/kg), le CRL 41 190 diminue nettement la durée de l'immobilité de la souris placée en immersion forcée.

M. *Conclusion:*

Il résulte des essais précédents que le CRL 41 190 présente vis-à-vis du SNC

1. des effets antidépresseurs:
   – antagonisme des hypothermies induites par la réserpine, l'oxotrémorine et l'apomorphine, et
   – diminution, aux doses élevées, de la durée de l'immobilité de la souris placée en immersion forcée;
2. un effet excitant discret se manifestant à forte dose:
   – hyperréactivité chez la souris,
   – diminution de la durée du sommeil barbiturique chez la souris,
   – augmentation modérée de l'agressivité intergroupes,
   – potentialisation des stéréotypies induites par l'amphétamine chez le rat, et
3. à forte dose:
   – une composante fruste de stimulation α-adrénergique objectivée par une diminution modérée du ptôsis réserpinique et une mydriase modérée chez la souris et le rat,
   – une aggravation des effets létaux de l'électrochoc.

En bref le CRL 41 190 agit essentiellement en tant que substance antidépressive du SNC. Le profil neuropsychopharmacologique du CRL 41 190 établi à partir des résultats obtenus chez l'animal rapproche ce produit des stimulants du type β-adrénergique. En effet l'absence d'activité anticholinergique périphérique et l'absence d'antagonisme net de l'immobilité de désespoir différencient le CRL 41 190 des antidépresseurs imipraminiques. Le CRL 41 190 se distingue par son action des antidépresseurs stimulants tels que l'amineptine ou même l'amphétamine. Enfin, l'absence d'antagonisme du ptôsis réserpinique et la rapidité d'apparition des effets du CRL 41 190 permettent de différencier le CRL 41 190 des inhibiteurs de la monoamine oxydase.

Des essais complémentaires ont été entrepris, le CRL 41 190 en solution dans de l'eau distillée étant administré par voie gastrique sous un volume de 20 ml/kg chez la souris mâle. On constate que le CRL 41 190 par voie gastrique, comme par voie intrapéritonéale, agit essentiellement en tant

qu'antidépresseur avec, à fortes doses, une composante éveillante et stimulante très modérée.

*Essais relatifs au CRL 41 214*

Le CRL 41 214 (produit de l'exemple 3) a été étudié selon les protocoles opératoires décrits ci-dessus pour le CRL 41 190.

A. *Toxicité*

Par voie I.P. chez la souris mâle, la DL-0 est supérieure à 256 mg/kg. Aux doses de 128 mg/kg et 256 mg/kg on observe une sédation et une dyspnée.

B. *Comportement global et réactivités*

On observe:

1. *Chez la souris:*
   - *aux doses de 2 à 32 mg/kg:* aucune modification nette du comportement et des réactivités;
   - *à la dose de 128 mg/kg:* une mydriase modérée pendant 3 h, et une sédation.
2. *Chez le rat:*
   - *aux doses de 1 et 4 mg/kg:* aucune modification nette du comportement, des réactivités, de la température rectale et du diamètre pupillaire par rapport au lot témoin;
   - *à la dose de 16 mg/kg:* une mydriase d'une durée de 2 h qui est maximale 0,5 h après administration, et
   - *à la dose de 64 mg/kg:* une diminution de la fréquence respiratoire pendant 0,5 h et une mydriase d'une durée de 3 h qui est maximale 1 h après administration.

C. *Interaction avec l'apomorphine*

1. *Chez la souris:*
   On observe dès la dose de 2 mg/kg, mais surtout aux doses de 32 mg/kg à 128 mg/kg, que le CRL 41 214 antagonise l'hypothermie induite par l'apomorphine.
   On constate que les comportements de verticalisation et les stéréotypies ne sont pratiquement pas modifiés.
2. *Chez le rat:*
   On observe que le CRL 41 214 laisse inchangées les stéréotypies induites par l'apomorphine.

D. *Interaction avec l'amphétamine*

On note que, aux doses de 4 mg/kg, 16 mg/kg et surtout à 64 mg/kg, le CRL 41 214 potentialise nettement les stéréotypies amphétaminiques.

E. *Interaction avec la réserpine*

On constate que, dès la dose de 8 mg/kg, mais surtout aux doses de 32 mg/kg et 128 mg/kg, le CRL 41 214 antagonise l'hypothermie réserpinique sans modifier le ptôsis.

F. *Interaction avec l'oxotrémorine*

1. *Action sur la température*
   Dès la dose de 8 mg/kg, mais surtout aux doses de 32 mg/kg et 128 mg/kg, le CRL 41 214 s'oppose à l'hypothermie induite par l'oxotrémorine.
2. *Action sur les tremblements*
   Aux doses de 32 mg/kg et 128 mg/kg, le CRL 41 214 diminue faiblement l'intensité des tremblements dus à l'oxotrémorine.
3. *Action sur les symptômes cholinergiques périphériques*
   Le CRL 41 214 ne modifie pratiquement pas les signes de stimulation cholinergique périphérique dus à l'oxotrémorine.

G. *Action sur le test des quatre plaques, la traction et l'électrochoc*

On observe que le CRL 41 214 n'entraîne pas d'augmentation nette du nombre de passages punis, qu'il ne provoque pas d'incapacité motrice majeure, qu'il ne modifie pratiquement pas les effets convulsivants, mais qu'il aggrave, à la dose de 128 mg/kg, les effets létaux de l'électrochoc.

H. *Action sur la motilité spontanée*

A la plus forte dose utilisée (128 mg/kg), le CRL 41 214 augmente nettement l'activité motrice spontanée de la souris.

I. *Action sur l'agressivité intergroupes*

Aux doses utilisées on observe que le CRL 41 214 ne modifie pratiquement pas le nombre de combats.

J. *Action vis-à-vis de quelques comportements perturbés par divers agents*

1. *Motilité réduite par habituation à l'enceinte*
   A la plus forte dose utilisée (128 mg/kg), le CRL 41 214 entraîne une reprise nette d'activité motrice chez la souris habituée à son enceinte.
2. *Motilité réduite par agression hypoxique*
   On constate que, à la plus forte dose utilisée (128 mg/kg), le CRL 41 214 semble entraîner une amélioration très modérée de la récupération motrice chez la souris dont la motilité a été déprimée à la suite d'un séjour bref dans une enceinte à pression réduite.
3. *Anoxie asphyxique*
   Le CRL 41 214 diminue très modérément le délai d'apparition des convulsions et de la mort consécutives à une anoxie asphyxique provoquée par un curarisant, le triiodoéthylate de gallamine.

K. *Interaction avec le barbital*

On observe que, à la plus forte dose utilisée (128 mg/kg), le CRL 41 214 diminue nettement la durée du sommeil barbiturique.

L. *Action sur le «désespoir comportemental»*

Aux doses de 32 et 128 mg/kg, le CRL 41 214 diminue très modérément la durée de l'immobilité de la souris placée en immersion forcée.

M. *Conclusion*

Les résultats des essais donnés ci-dessus montrent que le CRL présente:

1. des effets antidépresseurs:

– antagonisme des hypothermies induites par la réserpine, l'oxotrémorine et l'apomorphine,
– diminution aux doses élevées de la durée de l'immobilité de la souris placée en immersion forcée;

2. un effet excitant se manifestant à dose élevée:
– augmentation de l'activité motrice spontanée chez la souris,
– reprise de l'activité motrice chez la souris habituée à son enceinte,
– amélioration de la récupération motrice chez la souris placée en hypoxie aiguë,
– diminution de la durée du sommeil barbiturique chez la souris,
– potentialisation des stéréotypies induites par l'amphétamine chez le rat; et
3. à fortes doses:
– une composante de stimulation α-adrénergique objectivée par une mydriase chez la souris et le rat,
– un effet potentialisateur des effets létaux de l'électrochoc,
– une diminution très modérée du délai d'apparition des convulsions et de la mort consécutives à une anoxie asphyxique.

En bref le CRL 41 214 agit essentiellement sur le SNC en tant que substance antidépressive avec une composante excitante à forte dose. D'une manière générale les propriétés antidépressives se manifestent à des doses beaucoup plus faibles que l'effet excitant.

Le profil neuropsychopharmacologique du CRL 41 214 montre que ce produit se différencie des antidépresseurs imipraminiques par l'absence d'activité anticholinergique et l'absence d'antagonisme net de l'immobilité dite de «désespoir» (l'activité observée à la forte dose pouvant être en relation avec l'effet excitant), et qu'il se différencie des inhibiteurs de la monoamine oxydase par l'absence d'antagonisme du ptôsis réserpinique et par la rapidité d'apparition de ses effets.

Les CRL 41 250 (Ex. 9) et CRL 41 252 (Ex. 10) présentant des effets vasodilatateurs intéressants à côté de leurs propriétés antidépressives et sédatives, on a résumé ci-après les résultats des essais cardiovasculaires qui ont été entrepris de façon approfondie avec ces deux produits.

*Essais relatifs au CRL 41 250*

a) Quatre chiens (poids moyen: 13,8 kg) anesthésiés au nembutal reçoivent le CRL 41 250 par voie intraduodénale, aux doses successives de 0,1 mg/kg, 0,5 mg/kg, 1 mg/kg, 2,5 mg/kg, 5 mg/kg puis 10 mg/kg. On mesure la pression artérielle, la fréquence cardiaque, le débit artériel fémoral, le débit artériel vertébral, la température rectale et on observe la coloration de la peau.

On constate que le CRL 41 250 augmente le débit fémoral chez le chien anesthésié à la dose de 19,1 mg/kg I.D. et qu'il provoque une légère bradycardie à la dose de 1 mg/kg I.D.; on n'observe aucun effet sur la pression artérielle.

b) Sur les mêmes chiens on a étudié l'influence du produit vis-à-vis de l'isoprénaline et de la noradrénaline.

Vis-à-vis de l'isoprénaline (10 µg/kg I.V.), on constate que la dose cumulée de 19,1 mg/kg I.D. de CRL 41 250 ne modifie pas la pression artérielle diastolique ni la fréquence cardiaque: la pression artérielle diastolique passe de 148 à 47 mmHg (soit environ de $1,9 \times 10^4$ à $6,2 \times 10^3$ Pa) au lieu de 151 à 43 mmHg (soit de $2,0 \times 10^4$ à $5,7 \times 10^3$ Pa), et la fréquence cardiaque passe de 175 à 265 battements/minute au lieu de 180 à 271 battements/minute.

L'hypertension à la noradrénaline (2 µg/kg I.V.) est légèrement augmentée par le CRL 41 250 (à la dose cumulée de 19,1 mg/kg I.D.), la pression artérielle systolique passe de 191 à 330 mmHg (soit environ de $2,5 \times 10^4$ à $4,4 \times 10^4$ Pa) au lieu de 204 à 304 mmHg (soit environ de $2,7 \times 10^4$ à $4,0 \times 10^4$ Pa).

*Essais relatifs au CRL 41 252*

a) Deux chiens (poids moyen: 14,5 kg) reçoivent le CRL 41 252 par voie intraduodénale, aux doses successives de 0,1 mg/kg, 0,5 mg/kg, 1 mg/kg, 2,5 mg/kg, 5 mg/kg, 10 mg/kg et 20 mg/kg. On mesure la pression artérielle, la fréquence cardiaque, le débit artériel fémoral, le débit artériel vertébral, les températures rectale et cutanée et on observe la coloration de la peau.

On constate que le CRL 41 252 n'a pas d'effet sur la pression artérielle ni sur la fréquence cardiaque. Il augmente les débits fémoraux à partir de la dose de 10 mg/kg I.D., cet effet étant confirmé par l'injection I.M. de 10 mg/kg. On constate par ailleurs que le CRL 41 252 augmente les températures rectale et cutanée.

b) Sur les mêmes chiens on a étudié l'influence du produit vis-à-vis de l'isoprénaline et de la noradrénaline.

Les effets de l'isoprénaline testés après la dose cumulée de 39,1 mg/kg I.D. de CRL 41 252 sont légèrement diminués. Avec 10 µg/kg I.V. d'isoprénaline la pression artérielle diastolique du CRL 41 252 passe de 128 à 58 mmHg (soit environ $1,7 \times 10^4$ à $7,7 \times 10^3$ Pa) au lieu de 152 à 48 mmHg (soit environ de $2,0 \times 10^4$ à $6,4 \times 10^3$ Pa), et la fréquence cardiaque de 215 à 302 battements/minute au lieu de 192 à 310 battements/minute.

L'hypertension à la noradrénaline (2 µg/kg I.V.) est augmentée par le CRL 41 252 (à la dose cumulée de 39,1 mg/kg I.D.), la pression artérielle systolique passe de 208 à 352 mmHg (soit environ de $2,77 \times 10^4$ à $4,7 \times 10^4$ Pa) au lieu de 204 à 328 mmHg (soit environ de 2,71 à $4,3 \times 10^4$ Pa).

En clinique on a obtenu de bons résultats chez l'homme dans le traitement des dépressions par administration orale des CRL 41 190 et CRL 41 214, notamment sous forme de gélules ou comprimés renfermant chacun 25 mg ou 50 mg de principe actif à raison de 1 à 2 prises par jour pendant deux à trois semaines. On a également observé que les propriétés antidépressives du CRL 41 190 et du CRL 41 214 se manifestaient de façon très bénéfique chez les personne âgées.

Selon l'invention on préconise donc l'utilisation

d'une substance choisie parmi (i) les racémiques de formule I, (ii) leurs isomères énantiomères et (iii) les sels d'addition correspondants non toxiques, pour l'obtention d'un médicament antidépresseur du SNC destiné à une utilisation en thérapeutique humaine vis-à-vis des dépressions.

TABLEAU I

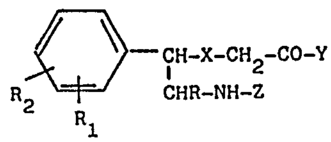

| Produit | N° de Code | X | Y | Z | R | $R_1$ | $R_2$ | Point de fusion (° C) |
|---|---|---|---|---|---|---|---|---|
| Ex. 1 (a) | CRL 41 190 | S | $NH_2$ | $C(CH_3)_3$ | H | H | H | 154-155 (b) |
| Ex. 2 (c) | CRL 41 191 | SO | $NH_2$ | $C(CH_3)_3$ | H | H | H | 147-148 |
| Ex. 3 (a) | CRL 41 214 | S | NHOH | $C(CH_3)_3$ | H | H | H | 198-200 (b) |
| Ex. 4 (d) | CRL 41 215 | S | $OCH_3$ | $CH(CH_3)_2$ | H | H | H | 138-139 |
| Ex. 5 (a) | CRL 41 216 | S | $NH_2$ | $CH(CH_3)_2$ | H | H | H | 118-120 |
| Ex. 6 (c) | CRL 41 225 | SO | $NH_2$ | $CH(CH_3)_2$ | H | H | H | 128-130 |
| Ex. 7 (c) | — | S | $OCH_3$ | $C(CH_3)_3$ | H | H | H | — |
| Ex. 8 (a) | — | S | NHOH | $CH(CH_3)_2$ | H | H | H | — |
| Ex. 9 (d) | CRL 41 250 | S | $NH_2$ | $C(CH_3)_3$ | $CH_3$ | H | H | 156-157 |
| Ex. 10 (d) | CRL 41 252 | S | $NH_2$ | $C(CH_3)_3$ | H | 2-F | H | 118 |
| Ex. 11 (d) | CRL 41 253 | S | $NH_2$ | $CH(CH_3)_2$ | H | 3-Cl | 4-Cl | 158 |
| Ex. 12 (c) | — | S | $OCH_3$ | $C(CH_3)_3$ | $CH_3$ | H | H | — |
| Ex. 13 (c) | — | S | $OCH_3$ | $C(CH_3)_3$ | H | 2-F | H | — |
| Ex. 14 (c) | — | S | $OCH_3$ | $CH(CH_3)_2$ | H | 3-Cl | 4-Cl | — |

Notes:
(a) = chlorhydrate
(b) = avec décomposition
(c) = base libre
(d) = fumarate

Diagramme A

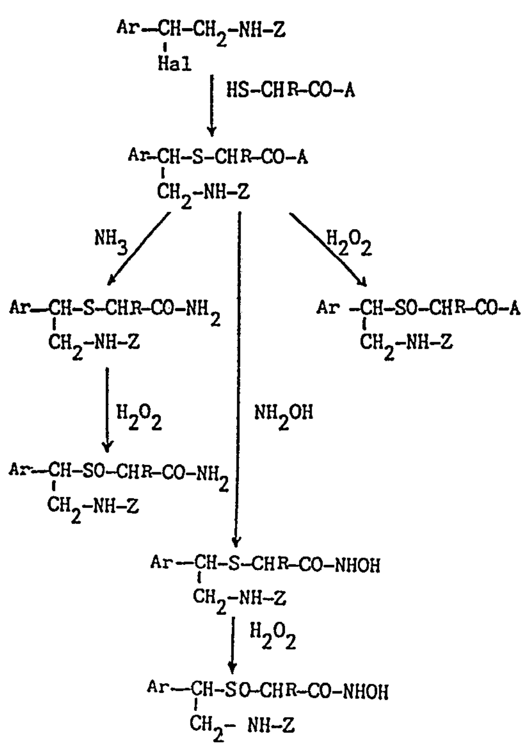


**Revendications** pour les Etats contractants BE, CH, DE, FR, GB, IT, LI, LU, NL, SE

1. Nouveau dérivé aminé d'acide (benzylthio)acétique, caractérisé en ce qu'il est choisi parmi l'ensemble constitué par:

a) les dérivés d'acide [α-(alkylaminométhyl)-benzylthio]-acétique de formule générale:

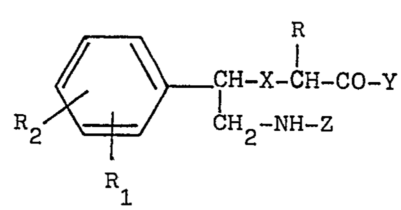 (I)

dans laquelle

X est S ou SO,

Y est un groupe alkoxy en $C_1$-$C_4$, un groupe $NH_2$ ou un groupe NHOH,

Z est un groupe alkyle en $C_1$-$C_4$,

R est H ou $CH_3$,

$R_1$ et $R_2$, identiques ou différents, représentent chacun H, F, Cl ou Br;

b) leurs isomères optiques, et

c) leurs sels d'addition.

2. Dérivé selon la revendication 1, caractérisé

en ce que X est S, Y est alkoxy en $C_1$-$C_4$, et Z est $CH(CH_3)_2$ ou $C(CH_3)_3$.

3. Dérivé selon la revendication 1, caractérisé en ce que X est S, Y est $NH_2$, et Z est $CH(CH_3)_2$ ou $C(CH_3)_3$.

4. Dérivé selon la revendication 1, caractérisé en ce que X est S, Y est NHOH, et Z est $CH(CH_3)_2$ ou $C(CH_3)_3$.

5. Dérivé selon la revendication 1, caractérisé en ce que X est SO, Y est $NH_2$ ou NHOH, et Z est $CH(CH_3)_2$ ou $C(CH_3)_3$.

6. Dérivé d'acide [α-(alkylaminométhyl)-benzylthio]-acétique, caractérisé en ce qu'il est choisi parmi l'ensemble constitué par:

(i) le racémique et les isomères énantiomères de [α-(tertiobutylaminométhyl)-benzylthio]-acétamide, et

(ii) leurs sels d'addition.

7. Dérivé d'acide [α-(alkylaminométhyl)-benzylthio]-acétique, caractérisé en ce qu'il est choisi parmi l'ensemble constitué par:

(i) le racémique et les isomères énantiomères de l'acide [α-(tertiobutylaminométhyl)-benzylthio]-acétohydroxamique, et

(ii) leurs sels d'addition.

8. Composition thérapeutique, caractérisée en ce qu'elle renferme, en association avec un excipient physiologiquement acceptable, au moins un dérivé d'acide [α-(alkylaminométhyl)-benzylthio]-acétique selon l'une quelconque des revendications 1 à 7.

9. Procédé de préparation d'un dérivé d'acide [α-(alkylaminométhyl)-benzylthio]-acétique de formule I selon la revendication 1, caractérisé en ce que:

a) l'on fait réagir une N-alkyl-N-(2-halogéno-2-phényléthyl)-amine de formule:

$Ar-CH-CH_2-NH-Z$ (II)
|
Hal

où Hal est un atome de fluor, de chlore ou de brome, Ar représente un groupe $(R_1R_2)C_6H_3$ dans lequel $R_1$ et $R_2$ sont définis comme indiqué ci-dessus, et Z est défini comme indiqué ci-dessus, avec un thioglycolate d'alkyle de formule:

$HS-CHR-CO-A$ (III)

où A est un groupe alkoxy en $C_1$-$C_4$ et R est H ou $CH_3$, dans un alcool de formule:

$H-A$ (IV)

où A est défini comme ci-dessus, en présence d'un métal alcalin, à une température comprise entre la température ambiante et la température de reflux du milieu réactionnel, pour obtenir un [α-(alkylaminométhyl)-benzylthio]-acétate d'alkyle de formule:

$Ar-CH-S-CHR-CO-A$ (V)
|
$CH_2-NH-Z$

b) si nécessaire on fait réagir, pendant au moins 5 h à une température comprise entre 15 et 25°C, le composé [α-(alkylaminométhyl)-benzylthio]-acétate d'alkyle ainsi obtenu avec un composé choisi parmi l'ensemble constitué par $NH_3$ et $NH_2OH$ pour obtenir l'amide de formule:

$Ar-CH-S-CHR-CO-NH_2$ (VI)
|
$CH_2-NH-Z$

et respectivement l'acide hydroxamique de formule:

$Ar-CH-S-CHR-CO-NHOH$ (VII)
|
$CH_2-NH-Z$

correspondants, et

c) si nécessaire, on soumet le dérivé soufré choisi parmi l'ensemble constitué par les composés de formule V, VI et VII à une réaction d'oxydation au moyen de $H_2O_2$, pour obtenir un dérivé sulfinyle de formule I selon la revendication 1 où X est SO.

10. Utilisation d'une substance choisie parmi (i) les racémiques de formule I selon la revendication 1, (ii) leurs isomères énantiomères et (iii) les sels d'addition correspondant non toxiques pour l'obtention d'un médicament antidépresseur du SNC destiné à une utilisation en thérapeutique humaine vis-à-vis des dépressions.

**Revendications** pour l'Etat contractant: AT

1. Procédé de préparation d'un dérivé d'acide [α-(alkylaminométhyl)-benzylthio]-acétique répondant à la formule générale:

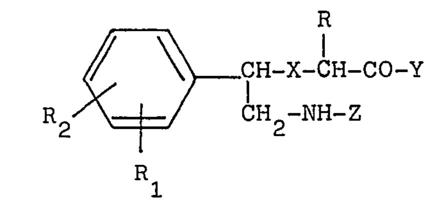
(I)

dans laquelle

X est S ou SO,

Y est un groupe alkoxy en $C_1$-$C_4$, un groupe $NH_2$ ou un groupe NHOH,

Z est un groupe alkyle en $C_1$-$C_4$,

R est H ou $CH_3$,

$R_1$ et $R_2$, identiques ou différents, représentent chacun H, F, Cl ou Br; ses isomères optiques et les sels d'addition correspondants, ledit procédé étant caractérisé en ce qu'il comporte les étapes suivantes:

a) l'on fait réagir une N-alkyl-N-(2-halogéno-2-phényl-éthyl)-amine de formule:

$Ar-CH-CH_2-NH-Z$ (II)
|
Hal

où Hal est un atome de fluor, de chlore ou de brome,

Ar représente un groupe $(R_1R_2)C_6H_3$ dans lequel $R_1$ et $R_2$ sont définis comme indiqué ci-dessus, et Z est défini comme indiqué ci-dessus, avec un thioglycolate d'alkyle de formule:

$$HS-CHR-CO-A \quad (III)$$

où A est un groupe alkoxy en $C_1$-$C_4$ et R est H ou $CH_3$, dans un alcool de formule:

$$H-A \quad (IV)$$

où A est défini comme ci-dessus, en présence d'un métal alcalin, à une température comprise entre la température ambiante et la température de reflux du milieu réactionnel, pour obtenir un [α-(alkylaminométhyl)-benzylthio]-acétate d'alkyle de formule:

$$Ar-\underset{\underset{CH_2-NH-Z}{|}}{CH}-S-CHR-CO-A \quad (V)$$

b) si nécessaire on fait réagir, pendant au moins 5 h à une température comprise entre 15 et 25°C, le composé [α-(alkylaminométhyl)-benzylthio]-acétate d'alkyle ainsi obtenu avec un composé choisi parmi l'ensemble constitué par $NH_3$ et $NH_2OH$ pour obtenir l'amide de formule:

$$Ar-\underset{\underset{CH_2-NH-Z}{|}}{CH}-S-CHR-CO-NH_2 \quad (VI)$$

et respectivement l'acide hydroxamique de formule:

$$Ar-\underset{\underset{CH_2-NH-Z}{|}}{CH}-S-CHR-CO-NHOH \quad (VII)$$

correspondants, et

c) si nécessaire, on soumet le dérivé soufré choisi parmi l'ensemble constitué par les composés de formule V, VI et VII à une réaction d'oxydation au moyen de $H_2O_2$, pour obtenir un dérivé sulfinyle de formule I où X est SO.

2. Procédé selon la revendication 1, caractérisé en ce que X est S, Y est alkoxy en $C_1$-$C_4$, et Z est $CH(CH_3)_2$ ou $C(CH_3)_3$.

3. Procédé selon la revendication 1, caractérisé en ce que X est S, Y est $NH_2$, et Z est $CH(CH_3)_2$ ou $C(CH_3)_3$.

4. Procédé selon la revendication 1, caractérisé en ce que X est S, Y est NHOH, et Z est $CH(CH_3)_2$ ou $C(CH_3)_3$.

5. Procédé selon la revendication 1, caractérisé en ce que que X est SO, Y est $NH_2$ ou NHOH, et Z est $CH(CH_3)_2$ ou $C(CH_3)_3$.

**Patentansprüche** für die Vertragsstaaten BE, CH, DE, FR, GB, IT, LI, LU, NL, SE

1. Neue (Benzylthio)-essigsäure-Derivate, dadurch gekennzeichnet, dass sie ausgewählt sind aus der durch

a) die Derivate der [α-(Alkylaminomethyl)-benzylthio]-essigsäure mit der allgemeinen Formel:

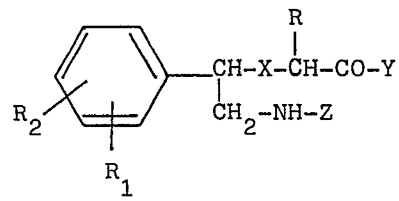

(I)

in welcher

X S oder SO ist,

Y eine $C_1$-$C_4$-Alkoxygruppe, eine $NH_2$-Gruppe oder eine NHOH-Gruppe darstellt,

Z eine $C_1$-$C_4$-Alkylgruppe ist,

R H oder $CH_3$ entspricht sowie

$R_1$ und $R_2$, gleich oder verschieden, jeweils H, F, Cl oder Br bedeuten,

b) deren optischen Isomeren, und

c) deren Additionssalzen gebildeteten Gruppierung.

2. Derivate nach Anspruch 1, dadurch gekennzeichnet, dass X S ist, Y einem $C_1$-$C_4$-Alkoxyrest entspricht und Z $CH(CH_3)_2$ oder $C(CH_3)_3$ darstellt.

3. Derivate nach Anspruch 1, dadurch gekennzeichnet, dass X S ist, Y $NH_2$ entspricht und Z $CH(CH_3)_2$ oder $C(CH_3)_3$ darstellt.

4. Derivat nach Anspruch 1, dadurch gekennzeichnet, dass X S ist, Y NHOH entspricht und Z $CH(CH_3)_2$ oder $C(CH_3)_3$ darstellt.

5. Derivate nach Anspruch 1, dadurch gekennzeichnet, dass X SO ist, Y $NH_2$ oder NHOH entspricht und Z $CH(CH_3)_2$ oder $C(CH_3)_3$ darstellt.

6. Derivate der [α-(Alkylaminomethyl)-benzylthio]-essigsäure, dadurch gekennzeichnet, dass sie ausgewählt sind aus der Gruppierung, die gebildet wird aus

(i) dem Racemat und den enantiomeren Isomeren vom [α-(tert.-Butylaminomethyl)-benzylthio]-acetamid, und

(ii) deren Additionssalzen.

7. Derivate der [α-(Alkylaminomethyl)-benzylthio]-essigsäure, dadurch gekennzeichnet, dass sie ausgewählt sind aus der Gruppierung, die gebildet wird aus

(i) dem Racemat und den enantiomeren Isomeren der [α-(tert.-Butylaminomethyl)-benzylthio]-acetohydroxamsäure, und

(ii) deren Additionssalzen.

8. Therapeutische Zusammensetzung, dadurch gekennzeichnet, das sie neben einer physiologisch annehmbaren Grundmasse wenigstens eines der Derivate der [α-(Alkylaminomethyl)-benzylthio]-essigsäure nach irgendeinem der Ansprüche 1 bis 7 enthält.

9. Verfahren zur Herstellung der [α-(Alkylaminomethyl)-benzylthio]-essigsäure-Derivate der Formel I gemäss Anspruch 1, dadurch gekennzeichnet, dass man

a) ein N-Alkyl-N-(2-halogeno-2-phenylethyl)-amin der Formel:

$$Ar-\underset{\underset{Hal}{|}}{CH}-CH_2-NH-Z \quad (II)$$

in welcher Hal einem Fluor-, Chlor- oder Brom-Atom entspricht, Ar die Gruppe $(R_1R_2)C_6H_3$ mit der obigen Bedeutung von $R_1$ und $R_2$ darstellt sowie Z die oben angeführte Bedeutung hat, mit einem Alkylthioglykolat der Formel:

HS−CHR−CO−A (III)

in welcher A eine $C_1$-$C_4$-Alkoxygruppe darstellt und R H oder $CH_3$ ist, in einem Alkohol der Formel:

H−A (IV)

mit A in der obigen Bedeutung in Gegenwart eines Alkalimetalls bei einer Temperatur im Bereich zwischen Raumtemperatur und Rückflusstemperatur der Reaktionsmischung zur Gewinnung eines [α-(Alkylaminomethyl)-benzylthio]-alkylacetats der Formel:

Ar−CH−S−CHR−CO−A (V)
|
$CH_2$−NH−Z

umsetzt,

b) gegebenenfalls wähend mindestens 5 Stunden bei einer Temperatur zwischen 15 und 25°C das wie unter a) erhaltene [α-(Alkylaminomethyl)-benzylthio]-alkylacetat mit $NH_3$ oder $NH_2OH$ weiterumsetzt zur Gewinnung des Amids der Formel:

Ar−CH−S−CHR−CO−$NH_2$ (VI)
|
$CH_2$−NH−Z

beziehungsweise der Hydroxamsäure mit der Formel:

Ar−CH−S−CHR−CO−NHOH (VII)
|
$CH_2$−NH−Z

und

c) gewünschtenfalls die schwefelhaltigen Verbindungen entsprechend einer der Formeln V, VI oder VII zwecks Gewinnung der Sulfoxidderivate gemäss der Formel I im Anspruch 1 mit der Bedeutung X = SO einer Oxydationsreaktion mittels $H_2O_2$ unterwirft.

10. Verwendung einer Substanz, die ausgewählt ist aus

(i) den Racematen der Formel I gemäss Anspruch 1,

(ii) deren enantiomeren Isomeren, und

(iii) deren nicht-toxischen Additionssalzen zur Herstellung eines in Bezug auf das SNC antidepressiv wirksamen Medikaments, das zur therapeutischen Verwendung beim Menschen gegenüber Depressionen bestimmt ist.

**Patentansprüche** für den Vertragsstaat: AT

1. Verfahren zur Herstellung von [α-(Alkylaminomethyl)-benzylthio]-essigsäure-Derivaten der allgemeinen Formel:

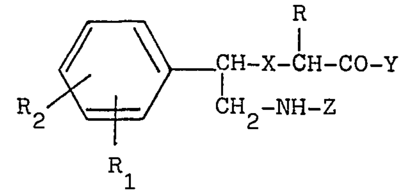

(I)

in welcher

X S oder SO ist,

Y eine $C_1$-$C_4$-Alkoxygruppe, eine $NH_2$-Gruppe oder eine NHOH-Gruppe darstellt,

Z eine $C_1$-$C_4$-Alkylgruppe ist,

R H oder $CH_3$ entspricht sowie

$R_1$ und $R_2$, gleich oder verschieden, jeweils H, F, Cl oder Br bedeuten,

deren optischen Isomeren und deren Additionssalzen, dadurch gekennzeichnet, dass man

a) ein N-Alkyl-N-(2-halogeno-2-phenylethyl)-amin der Formel:

Ar−CH−$CH_2$−NH−Z (II)
|
Hal

in welcher Hal einem Fluor-, Chlor- oder Brom-Atom entspricht, Ar die Gruppe $(R_1R_2)C_6H_3$ mit der obigen Bedeutung von $R_1$ und $R_2$ darstellt sowie Z die oben angeführte Bedeutung hat, mit einem Alkylthioglykolat der Formel:

HS−CHR−CO−A (III)

in welcher A eine $C_1$-$C_4$-Alkoxygruppe darstellt und R H oder $CH_3$ ist, in einem Alkohol der Formel:

H−A (IV)

mit A in der obigen Bedeutung in Gegenwart eines Alkalimetalls bei einer Temperatur im Bereich zwischen Raumtemperatur und Rückflusstemperatur der Reaktionsmischung zur Gewinnung eines [α-(Alkylaminomethyl)-benzylthio]-alkylacetats der Formel:

Ar−CH−S−CHR−CO−A (V)
|
$CH_2$−NH−Z

umsetzt,

b) gegebenenfalls während mindestens 5 Stunden bei einer Temperatur zwischen 15 und 25°C das wie unter a) erhaltene [α-(Alkylaminomethyl)-benzylthio]-alkylacetat mit $NH_3$ oder $NH_2OH$ weiterumsetzt zur Gewinnung des Amids der Formel:

Ar−CH−S−CHR−CO−$NH_2$ (VI)
|
$CH_2$−NH−Z

beziehungsweise der Hydroxamsäure mit der Formel:

Ar−CH−S−CHR−CO−NHOH (VII)
|
$CH_2$−NH−Z

und

c) gewünschtenfalls die schwefelhaltigen Verbindungen entsprechend einer der Formeln V, VI oder VII zwecks Gewinnung der Sulfoxidderivate gemäss der Formel I im Anspruch 1 mit der Bedeutung X = SO einer Oxydationsreaktion mittels $H_2O_2$ unterwirft.

2. Verfharen nach Anspruch 1, dadurch gekennzeichnet, dass X S ist, Y einem $C_1$-$C_4$-Alkoxyrest entspricht und Z $CH(CH_3)_2$ oder $C(CH_3)_3$ darstellt.

3. Verfahren nach Anspruch 1, dadurch gekennzeichnet, dass X S ist, Y $NH_2$ entspricht und Z $CH(CH_3)_2$ oder $C(CH_3)_3$ darstellt.

4. Vefahren nach Anspruch 1, dadurch gekennzeichnet, das X S ist, Y NHOH entspricht und Z $CH(CH_3)_2$ oder $C(CH_3)_3$ darstellt.

5. Verfahren nach Anspruch 1, dadurch gekennzeichnet, dass X SO ist, Y $NH_2$ oder NHOH entspricht und Z $CH(CH_3)_2$ oder $C(CH_3)_3$ darstellt.

**Claims** for the contracting States BE, CH, DE, FR, GB, IT, LI, LU, NL, SE

1. An amine derivative of (benzylthio)acetic acid, characterized in that it is selected from the group consisting of

a) [α-(alkylaminomethyl)-benzylthio]-acetic acid derivatives of the general formula:

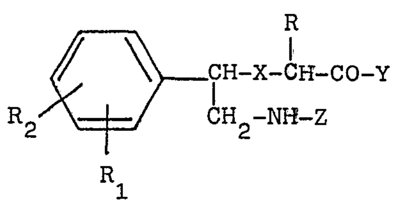

(I)

wherein

X is S or SO,

Y is $C_1$-$C_4$-alkoxy, $NH_2$ or NHOH,

Z is $C_1$-$C_4$-alkyl,

R is H or $CH_3$,

$R_1$ and $R_2$, which may be the same or different, represent each H, F, Cl or Br;

b) their optical isomers, and

c) addition salts thereof.

2. A derivative according to claim 1, characterized in that X is S, Y is $C_1$-$C_4$-alkoxy, and, Z is $CH(CH_3)_2$ or $C(CH_3)_3$.

3. A derivative according to claim 1, characterized in that X is S, Y is $NH_2$, and, Z is $CH(CH_3)_2$ or $C(CH_3)_3$.

4. A derivative according to claim 1, characterized in that X is S, Y is NHOH, and, Z is $CH(CH_3)_2$ or $C(CH_3)_3$.

5. A derivative according to claim 1, characterized in that X is SO, Y is $NH_2$ or NHOH, and, Z is $CH(CH_3)_2$ or $C(CH_3)_3$.

6. A derivative of [α-(alkylaminomethyl)-benzylthio]-acetic acid characterized in that it is selected from the group consisting of

(i) the racemate and enantiomer isomers of the [α-(t.-butylaminomethyl)-benzylthio]-acetamide, and

(ii) addition salts thereof.

7. A derivative of [α-(alkylaminomethyl)-benzylthio]-acetic acid, characterized in that it is selected from the group consisting of

(i) the racemate and enantiomer isomers of the [α-(t.-butylaminomethyl)-benzylthio]-acetohydroxamic acid, and

(ii) addition salts thereof.

8. A therapeutical composition, characterized in that it comprises, in association with a physiologically acceptable excipient, at least one [α-(alkylaminomethyl)-benzylthio]-acetic acid derivative according to any one of claims 1-7.

9. A method for preparing a [α-(alkylaminomethyl)-benzylthio]-acetic acid derivative of the formula I according to claim 1, said method comprising

a) reacting a N-alkyl-N-(2-halogeno-2-phenylethyl)-amine of the formula:

$$\begin{array}{l} Ar-CH-CH_2-NH-Z \\ \quad\;\; | \\ \quad\;\; Hal \end{array} \qquad (II)$$

wherein Hal represents F, Cl or Br, Ar represents a $(R_1R_2)C_6H_3$ group in which $R_1$ and $R_2$ are defined as indicated above, and Z is defined as indicated above, with an alkyl thioglycolate of the formula:

$$HS-CHR-CO-A \qquad (III)$$

wherein A is $C_1$-$C_4$-alkoxy and R is H or $CH_3$, in an alcohol of the formula:

$$H-A \qquad (IV)$$

wherein A is defined as indicated above, in the presence of an alcaline metal, at a temperature comprised between room temperature and the reflux temperature of the reaction medium, for obtaining an alkyl [α-(alkylaminomethyl)-benzylthio]-acetate of the formula:

$$\begin{array}{l} Ar-CH-S-CHR-CO-A \\ \quad\;\; | \\ \quad\;\; CH_2-NH-Z \end{array} \qquad (V)$$

b) if needed, reacting, for at least 5 h at a temperature between 15 and 25°C, said alkyl [α-(alkylaminomethyl)-benzylthio]-acetate thus obtained with a compound selected from $NH_3$ and $NH_2OH$ for obtaining respectively the corresponding amide of the formula

$$\begin{array}{l} Ar-CH-S-CHR-CO-NH_2 \\ \quad\;\; | \\ \quad\;\; CH_2-NH-Z \end{array} \qquad (VI)$$

and hydroxamic acid of the formula:

$$\begin{array}{l} Ar-CH-S-CHR-CO-NHOH \\ \quad\;\; | \\ \quad\;\; CH_2-NH-Z \end{array} \qquad (VII)$$

and

c) if needed, subjecting the sulfide compounds selected from the group consisting of compounds of the formulae V, VI and VII, to an oxidation reaction with $H_2O_2$ in order to obtain a sulfinyl compound of the formula I according to claim 1 in which X is SO.

10. The use of a substance selected from the group consisting of (i) racemates of the formula I according to claim 1, (ii) their enantiomer isomers, and (iii) non-toxic addition salts thereof, for obtaining a CNS-antidepressive medicament destined to be used in human therapy against depressions.

**Claims** for the contracting State: AT

1. A method for preparing a [α-(alkylaminomethyl)-benzylthio]-acetic acid derivative of the formula:

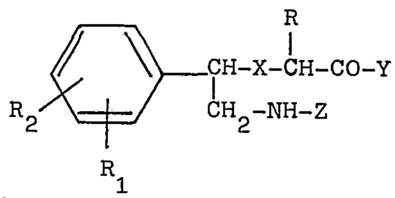 (I)

wherein

X is S or SO,

Y is $C_1$-$C_4$-alkoxy, $NH_2$ or NHOH,

Z is $C_1$-$C_4$-alkyl,

R is H or $CH_3$,

$R_1$ and $R_2$, which may be the same or different, represent each H, F, Cl or Br; its optical isomers and addition salts thereof, said method being characterized in that it comprises the following steps:

a) reacting a N-alkyl-N-(2-halogeno-2-phenylethyl)-amine of the formula:

$$\begin{array}{l} Ar-CH-CH_2-NH-Z \\ \quad\;\; | \\ \quad\;\; Hal \end{array} \qquad (II)$$

wherein Hal represents F, Cl or Br, Ar represents a $(R_1R_2)C_6H_3$ group in which $R_1$ and $R_2$ are defined as indicated above, and Z is defined as indicated above, with an alkyl thioglycolate of the formula

$$HS-CHR-CO-A \qquad (III)$$

wherein A is $C_1$-$C_4$-alkoxy and R is H or $CH_3$, in an alcohol of the formula:

$$H-A \qquad (IV)$$

wherein A is defined as indicated above, in the presence of an alcaline metal, at a temperature comprised between room temperature and the reflux temperature of the reaction medium, for obtaining an alkyl [α-(alkylaminomethyl)-benzylthio]-acetate of the formula:

$$\begin{array}{l} Ar-CH-S-CHR-CO-A \\ \quad\;\; | \\ \quad\;\; CH_2-NH-Z \end{array} \qquad (V)$$

b) if needed, reacting, for at least 5 h at a temperature of between 15 and 25°C, said alkyl [α-(alkylaminomethyl)-benzylthio]-acetate thus obtained with a compound selected from $NH_3$ and $NH_2OH$ for obtaining, respectively, the corresponding amide of the formula:

$$\begin{array}{l} Ar-CH-S-CHR-CO-NH_2 \\ \quad\;\; | \\ \quad\;\; CH_2-NH-Z \end{array} \qquad (VI)$$

and hydroxamic acid of the formula:

$$\begin{array}{l} Ar-CH-S-CHR-CO-NHOH \\ \quad\;\; | \\ \quad\;\; CH_2-NH-Z \end{array} \qquad (VII)$$

and

c) if needed, subjecting the sulfide compound selected from the group consisting of compounds of the formulae V, VI and VII, to an oxidation reaction with $H_2O_2$ in order to obtain a sulfinyl compound of the formula I in which X is SO.

2. A method according to claim 1, characterized in that X is S, Y is $C_1$-$C_4$-alkoxy, and, Z is $CH(CH_3)_2$ or $C(CH_3)_3$.

3. A method according to claim 1, characterized in that X is S, Y is $NH_2$, and Z is $CH(CH_3)_2$ or $C(CH_3)_3$.

4. A method according to claim 1, characterized in that X is S, Y is NHOH, and, Z is $CH(CH_3)_2$ or $C(CH_3)_3$.

5. A method according to claim 1, characterized in that X is SO, Y is $NH_2$ or NHOH, and, Z is $CH(CH_3)_2$ or $C(CH_3)_3$.